# EUROPÄISCHE PATENTANMELDUNG

(11) **EP 0 000 128 A1**
(43) Veröffentlichungstag der Anmeldung: **10.01.1979**
(21) Anmeldenummer: 78100118.5
(22) Anmeldetag: 08.06.1978
(51) Int. Cl.: C07D 307/80, C07D 209/12, C07D 333/56, A61K 31/34, A61K 31/38, A61K 31/40

(54) **Sulfamoyl-Arylketone und Verfahren zu ihrer Herstellung sowie ihre Verwendung als Arzneimittel**

(30) Priorität: 21.06.1977 DE 2727802
(71) Anmelder: HOECHST AKTIENGESELLSCHAFT, 65926 Frankfurt am Main (DE)
(72) Erfinder: Lang, Hans Jochen, Dr., D-6238 Hofheim am Taunus (DE); Musil, Josef, Dr., D-7815 Kirchzarten-Burg (DE); Muschaweck, Roman, Dr., D-6000 Frankfurt/Main (DE)

(57) **Zusammenfassung**

Heterocyclische 3-Sulfamoylarylketone, der allgemeinen Formel 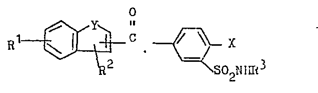 worin R¹ Wasserstoff, Halogen, Alkyl mit 1 bis 3 C-Atomen, Methoxy oder Aethoxy, R² und R³ gleich oder verschieden, Wasserstoff oder Alkyl mit 1 bis C 4-Atomen,X ein Halogenatom, Methyl oder Trifluormethyl und Y Sauerstoff, Schwefel oder NR⁴ bedeuten, wobei R⁴ für Wasserstoff oder Alkyl mit 1 bis 4 C-Atomen steht, mit urikosurischer, hypourikämischer und salidiuretischer Wirkung und ein Verfahren zur Herstellung dieser Verbindungen sowie pharmazeutische Präparate, die diese Verbindungen enthalten.

## Beschreibung

Gegenstand der Erfindung sind Verbindungen der allgemeinen Formel I 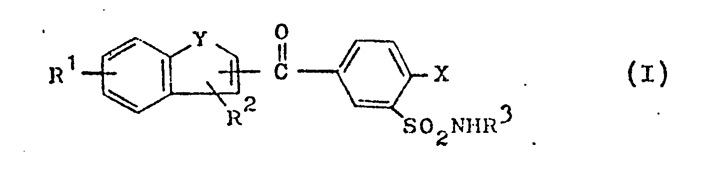 worin R¹ Wasserstoff, Halogen, Alkyl mit 1 bis 3 C-Atomen, Methoxy oder Äthoxy, R² und R³ gleich oder verschieden sein können und Wasserstoff oder Alkyl mit 1 bis 4 C-Atomen bedeutet, X ein Halogenatom, Methyl oder Trifluormethyl und Y Sauerstoff, Schwefel oder NR⁴ bedeuten, wobei R⁴ für Wasserstoff oder Alkyl mit 1 bis 4 C-Atomen steht.

Gegenstand der Erfindung ist weiterhin ein Verfahren zur Herstellung von Verbindungen der Formel I, das dadurch gekennzeichnet ist, dass man
a) Verbindungen der allgemeinen Formel II 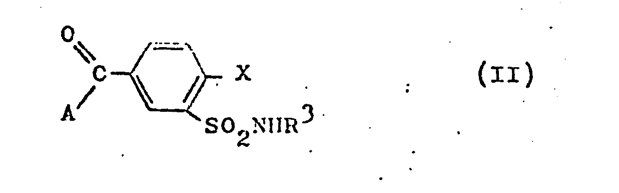 worin _{R}³ und X die angegebene Bedeutung besitzen und A für Halogen, Acyloxy, Hydroxy oder N(R)₂ steht, wobei R Wasserstoff, niederes Alkyl oder Phenyl ist; mit einer heterocyclischen Verbindung der allgemeinen Formel III 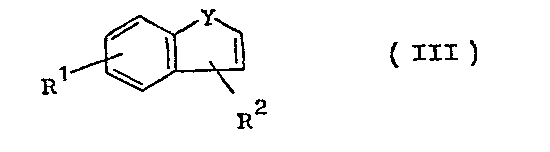 worin R¹, R² und Y die angegebene Bedeutung besitzen, in Gegenwart einer Lewis-Säure oder einer Protonensäure umsetzt,
b) Verbindungen der allgemeinen Formel IV 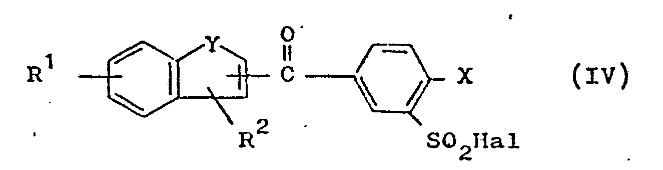 mit einem Amin der allgemeinen Formel V 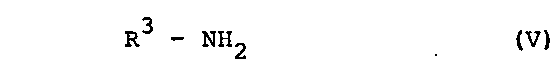 umsetzt, wobei R¹ bis R³, X und Y die angegebene Bedeutung besitzen und Hal für Halogen steht,
c) Verbindungen der allgemeinen Formel VI 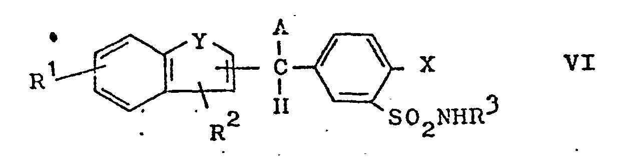 worin R¹ bis R³, A, X und Y die angegebene Bedeutung besitzen, mit einem Oxidationsmittel behandelt,
d) Verbindungen der allgemeinen Formel VII 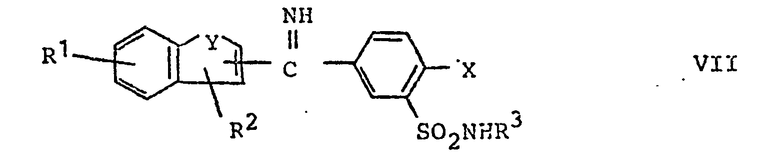 worin R¹ bis R³, X und Y die obige Bedeutung haben, gegebenenfalls in Form ihrer Säureadditionssalze der Hydrolyse unterwirft,
e) Verbindungen der allgemeinen Formel VIII 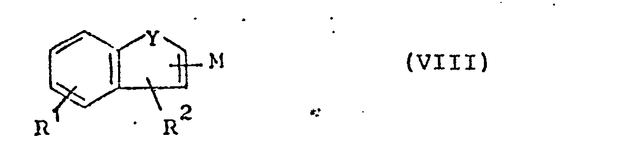 worin R bis ^{R2} die angegebene Bedeutung besitzen, M für Li, Mg Hal, Cd Hal, Hg Hal oder HgOCOCH₃ steht, und Y neben der angegebenen Bedeutung auch diejenige von NM besitzen kann, mit Verbindungen der allgemeinen Formel II umsetzt, worin A neben der angegebenen Bedeutung auch für einen 2-Mercaptopyridylrest kann, oder 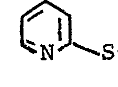 stehen
f) Verbindungen der allgemeinen Formel IX 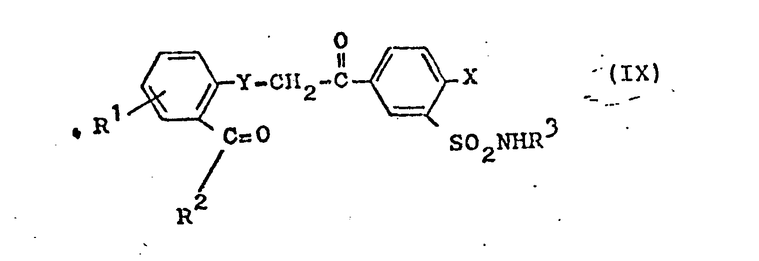 cyclisiert
   und gegebenenfalls die nach a) - f) erhaltenen Verbindungen der Formel I, in der R³ für Wasserstoff steht, anschliessend alkyliert.

Die Alkylreste für R ¹ bis R⁴ können geradkettig oder verzweigt sein.

Die unter a) bezeichnete Verfahrensweise wird so ausgeführt, dass man Verbindungen II mit den Verbindungen III bevorzugt im molaren Verhältnis 1 : 1 umsetzt, wobei die Reaktionsdurchführung hinsichtlich Katalysator, Reaktionstemperatur, Reaktionsdauer, Lösungsmittel und Aufarbeitung gemäss der Vorschrift für vergleichbare Beispiele z.B. nach Houben-Weyl, "Methoden der Organischen Chemie", 4. Auflage, Band 7/2 a, Seiten 15 - 375 (1973), erfolgt.

Wenn A für Hal oder Acyloxy steht, haben sich unter den für Friedel-Crafts-Reaktionen verwendeten Lösungsmitteln neben den in der Literatur beschriebenen üblichen Lösungsmitteln u.a. halogensubstituierte Benzolderivate, wie z.B. Fluor-, Difluor-, Chlor- oder Dichlorbenzol als besonders geeignet erwiesen. Als Friedel-Krafts-Katalysatoren, die sowohl Lewis-Säuren wie Protonensäuren umfassen, kommen bevorzugt Aluminiumchlorid, Zinn- und Titantetrachlorid zur Anwendung, wobei jedoch auch andere Katalysatoren, wie beispielsweise HF, BF₃, ZnCl₂, GaCl₃, J₂ verwendet werden können. Als Protonensäuren kommen z.B. HF, HClO₄ oder Polyphosphorsäure in Betracht. Insbesondere bei Anwendung von Aluminiumchlorid wie auch bei den anderen Katalysatoren muss berücksichtigt werden, dass die Sulfamoylfunktion mindestens 1 Mol AlCl₃ bzw. Lewis-Säure infolge Komplexbildung desaktiviert, also ein überschuss von mindestens 2 Mol an Lewis-Säure eingesetzt wird.

Wenn A für Hydroxy steht, verwendet man bevorzugt Aluminiumchlorid, Zinkchlorid, Bortrifluorid, aber auch Fluorwasserstoff und Perchlorsäure, sowie Polyphosphorsäure oder Phosphoroxychlorid als Katalysatoren, dagegen wird POCl₃ als katalysierende Lewis-Säure insbesondere bei Umsetzungen von III mit den Säureamid-Derivaten der Formel II, bei de-. nen Y für -NR₂ steht, vorteilhaft verwendet.

Nach Verfahrensweise b) bringt man Sulfochloride der Formel IV mit Ammoniak oder einem Amin in an sich bekannter Weise zur Reaktion, vergl. Houben-Weyl, "Methoden der Organischen Chemie", 4. Auflage, Band 9 (1955), Seiten 605-627.

Als Reaktionsmedien erwiesen sich polare Lösungsmittel,, wie Wasser, niedere Alkohole mit 1 bis 5 C-Atomen, Dioxan, Tetrahydrofuran, Dimethylacetamid, Mono-, Di- oder Tri- äthylenglykoldimethyläther als besonders geeignet, wobei man die Reaktion zwischen 0 und 100° C, bevorzugt zwischen 10 und 50° C durchführt. Die Reaktionsdauer liegt zwischen 1/2 und 70 Stunden, bevorzugt bei 4 bis 14 Stunden.

Die Sulfochloride der Formel IV können in an sich bekannter Weise auf verschiedenen Wegen erhalten werden. Bevorzugt erhält man sie durch Meerwein-Reaktion (Chem. Ber. 90, 841 (1957)) aus den Aminoderivaten der Formel X 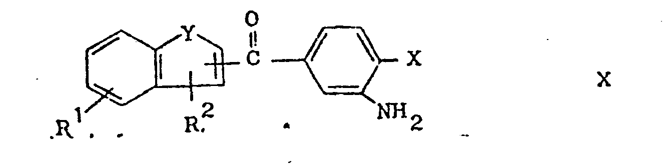 worin die Substituenten die angegebene Bedeutung haben.

Die Verbindungen der Formel X werden beispielsweise aus Verbindungen XI 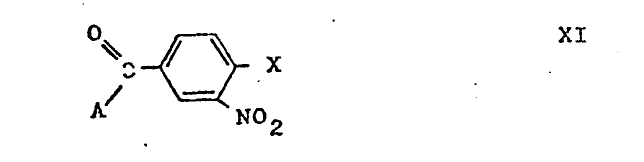 worin X und A die angegebene Bedeutung besitzen, durch -eine der Verfahrehsweise a) analoge Reaktion mit Verbindungen III und anschliessender Reduktion der Nitroverbindung XII 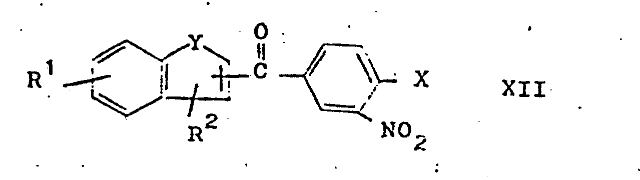 dargestellt.

Gemäss Verfahrensweise c) werden Verbindungen der allgemeinen Formel VI mit einem Oxidationsmittel in die Verbindungen der Formel I übergeführt. Es eignen sich sowohl organische wie auch anorganische Oxidationsmittel wie beispielsweise Salze und Komplexverbindungen des Fe⁺³, Nickelperoxid, Kaliumpermanganat, Chrom-VI-verbindungen, Kupfer-II-salze, Halogen, Stickoxide wie N₂0₃ in situ oder N0₂, HNO₃, Sauerstoff, anorganische und organische Peroxoverbindungen wie H₂0₂, Behzoper- und m-Chlorbenzopersäure, N-Chlor- und N-Brom-succinimid, Dimethylsulfoxid, aliphatische Nitroverbindungen, Ketone in Gegenwart eines Aluminiumalkoholates im Sinine einer Oppenauer-Oxidation. Hierbei hält man sich in Durchführung und Aufarbeitung an vergleichbare,in der Literatur erwähnte Beispiele, z.B. Houben-Weyl, "Methoden der Organischen Chemie", 4. Auflage, Band 4/1 b, (1975), Seiten 425, 465, 67.3, 901, und Band 7/2 a, (1973), Seiten 677 - 788. Wenn A eine OH-Gruppe bedeutet, hat sich als mildes und besonders geeignetes Oxidationsmittel aktives Mangan-IV-oxid erwiesen (vergl. z.B. A. J. Fatiadi, Synthesis 1976, 65; DE-OS 2 436 263), wobei man als Lösungsmittel vorzugsweise Acetonitril oder halogenierte Kohlenwasserstoffe, wie z.B. Methylenchlorid, Chloroform, Tetrachloräthan verwendet und die Reaktion bei Temperaturen zwischen 0° und 40° C, vorzugsweise zwischen 20° und 30° C, über eine Dauer von 6 bis 60 Stunden durchführt.

Zu den Verbindungen VI mit A = OH gelangt man in üblicher Weise z.B. durch Addition eines Aldehyds der allgemeinen Formel XIII 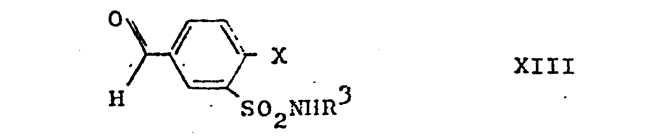 an Verbindungen der allgemeinen Formel III oder VIII.

Bei der Cxiäation von Verbindungen VI, worin A Acyloxy bedeutet, wendet man bevorzugt Chromsäure an, vergl. Org. Synth. 42, 79 (1962). Wenn A für Halogen steht, verwendet man neben Chromsäure bzw. Natriumbichromat i.m sauren Medium bevorzugt Dimethylsulfoxid, die Oxide tertiärer Amine wie z.B. Pyridin-N-oxid oder Trimethylaminoxid und aliphatische Nitroverbindungen wie z.B. 2-Nitropropan. Die Verbindungen VI, worin A für N(R)₂ steht, werden bevorzugt in die Verbindungen I übergeführt, vergl. Org. Prep. Proced. Int. 8, 33 (1976); J. Am. Chem. Soc. 97, 5927 (1975).

Man erhält die Verbindungen VI, worin A Halogen oder Acyloxy bedeutet, z.B. aus den entsprechenden Verbindungen mit A = OH durch Acylierung oder Chlorierung, wenn A eine N(R)₂-Gruppe bedeutet, beispielsweise aus den entsprechenden Verbindungen mit A = Halogen durch Umsetzung mit Aminen in üblicher Weise.

Gemäss Verfahrensweise d) werden Ketimine der Formel VII, die auch in Form ihrer Säureadditionssalze vorliegen können, hydrolysiert. Zur Herstellung der Verbindungen der Formel VII bringt man die Nitrile der Formel XIV 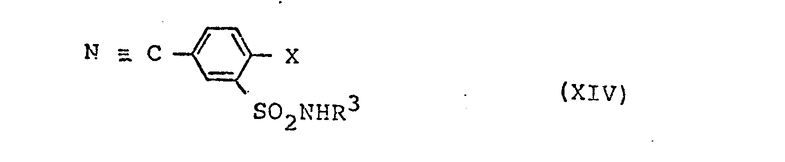 mit den Verbindungen der Formel III im Sinne einer Houben-Hoesch-Reaktion zur Umsetzung, vergl. Organic Reactions 5, 387 (1949). Dabei werden die beiden Reaktionspartner vorzugsweise im molaren Verhältnis 1 : 1 in einem inerten polaren und möglichst wasserfreien organischen Lösungsmittel, wie Diäthyläther, Diisopropyläther, Tetrahydrofuran, Eisessig, Dioxan, einem Halogenbenzol unter Bevorzugung von Chlorbenzol, zur Reaktion gebracht, vorteilhaft unter Verwendung von Mono-, Di- ider Triäthylenglykoldimethyl-oder diäthyläther als Lösungsmittel. In das Reaktionsgemisch leitet man über einen Zeitraum von 2 bis 20 Stunden einen trockenen Strom HCI-Gas bis zur Sättigung ein, bei Temperaturen zwischen -30° und +40° C, vorteilhaft zwischen -5° und +15° C. Anschliessend lässt man vorteilhaft das Gemisch 1 bis 3 Tage bei -5° bis +15° C stehen. Es kann auch in Gegenwart einer zusätzlichen Lewis-Säure, wieinsbesondere wasserfreiem Zink- oder Aluminiumchlorid gearbeitet werden.

Um Verunreinigungen abzutrennen, empfiehlt es sich, die intermediär entstehenden Ketimin-hydrochloride der Formel VII zu isolieren, obwohl prinzipiell auch eine Hydrolyse ohne weitere Isolierungs- und Reinigungsoperationen möglich ist. Die Ketimin-hydrochloride werden durch Zusatz eines weniger polaren Lösungsmittels durch Ausfällung, erhalten, insbesondere durch Diisopropyläther, Äther, aber auch Essigsäureniederalkylester, Aceton sowie Gemische der angegebenen Lösungsmittel mit Petroläther oder Cyclohexan.

Die Hydrolyse des Ketimin-hydrochlorides kann sowohl im alkalischen wie sauren Medium durchgeführt werden, wobei man die Verbindungen VII in Wasser oder Äthanol-Wasser-Gemischen gegebenenfalls in Gegenwart geringer Mengen an Ammoniak, Natronlauge, Calciumcarbonat, verdünnter Salzsäure oder Schwefelsäure erhitzt und das sich bildende Keton abfiltriert oder nach Extraktion mit einem organischen Lösungsmittel, bevorzugt mit Essigester, isoliert.

Die Ketimine der Formel VII können auch durch Umsetzung von Verbindungen der Formel VIII mit den Nitrilen der Formel XIV nach der Methode von Blaise, vergl. Houben-Weyl, 4. Aufl., Band 7/2 a, Seite 603 (1973), erhalten werden.

Als Lösungsmittel verwendet man die für metallorganische Reaktionen üblichen inerten und wasserfreien Lösungsmittel, bevorzugt Äther wie Diäthyläther, Dibutyläther, besonders vorteilhaft aber Tetrahydrofuran oder Mono-, Di-, Tri- äthylenglykoldimethyl- oder -diäthyläther. Aucr inerte aromatische Kohlenwasserstoffe wie Toluol oder Xylol können verwendet werden. Pro Mol XIV müssen, wenn R³ nicht Wasserstoff bedeutet, mindestens 2, bei R³ = H mindestens 3 Mol der Verbindungen VIII eingesetzt werden. Man arbeitet bevorzugt zwischen 30° und 130° C, zwischen 3 und 50 Stunden, meist wird das Reaktionsgemisch nach 10 bis 24stündigem Rühren durch Zersetzung mit Wasser aufgearbeitet. Die dabei erhaltenen Imine werden im sauren oder basischen Milieu in die Verbindungen der Formel I durch Hydrolyse überführt.

Gemäss Verfahrensweise e) bringt man Verbindungen der allgemeinen Formel II mit metallorganischen Verbindungen VIII zur Reaktion, wobei insbesondere die entsprechende Lithium-und Magnesium-organischen Verbindungen eingesetzt werden. Bei dieser Verfahrensweise kann A in den Verbindungen der Formel II auch den Mercaptopyridylrest bedeuten, vergl. Bull. Chem. Soc. Japan 47, 1777 (1974).

Dabei verwendet man pro Mol der Verbindungen II etwa 2 bis 2,5 Mole der Verbindung VIII, wenn R³ nicht für Wasserstoff steht, und etwa 3 bis 3,5 Mole VIII, wenn R³ Wasserstoff bedeutet. Die Umsetzung wird in einem für metallorganische Reaktionen üblichen inerten und wasserfreien Lösungsmittel, vorzugsweise in Äther, Tetrahydrofuran, Dioxan oder in einem Mono-, Di- oder Triäthylenglykoldimethyl- oder -diäthyläther durchgeführt, bevorzugt bei Temperaturen zwischen -0100° und +100° C. Nach Beendigung der Umsetzung werden die Reaktionsprodukte in üblicher Weise hydrolysiert, indem man beispielsweise das Reaktionsgemisch bei Temperaturen zwischen -5° und +20° C unter Aufrechterhaltung eines pH-Bereiches von 6 bis 11 in eine wässrige gesättigte Ammoniumchlorid-Lösung einträgt.

Die in Verfahrensweise e) bevorzugt verwendeten Verbindungen der Formel VIII mit M = Li oder Mg Hal gewinnt man dadurch, dass man auf Halogenverbindungen der Formel XIV 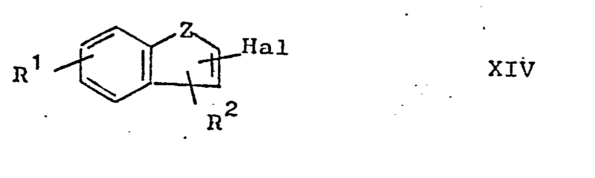 oder auf Verbindungen III das Metall M oder besonders vorteilhaft metallorganische Verbindungen R - M mit M = Mg Hal oder Li in bekannter Weise zur Einwirkung bringt (Houben-Weyl, 4. Auflage, Band XIII/1 (1970), Seiten 93 - 157, und Band XIII/2 a (1973), Seiten 54 - 152).

Gemäss Verfahrensweise f) werden Verbindungen der allgemeinen Formel IX unter üblichen Bedingungen cyclisiert (siehe z.B. Advances Het. Chem., Band 18, Seite 338 (1975), - oder Band 11, Seite 178 (1970). Die Cyclokondensation kann sowohl sauer wie alkalisch katalysiert werden; letzteres ist bevorzugt. Man arbeitet vorteilhaft mit den Alkalisalzen schwacher Säuren, beispielsweise mit dem Salz einer schwachen organischen Säure wie Natrium- oderKaliumacetat in Eisessig als geeignetem Lösungsmittel, oder mit Natrium- bzw. Kaliumcarbonat, Natrium- oder Kaliumalkoholaten wie Äthylat oder Methylat sowie mit Metallhydroxiden z.B. NaOH oder KOH in einem geeigneten polaren organischen Lösungsmittel, wie Aceton, Methyläthylketon, Dimethylformamid, Dimethylacetamid oder niedere Alkohole mit 1 bis 4 C-Atomen oder Gemischen der angegebenen Lösungsmittel. Man arbeitet über 1/2 bis 12 Stunden bei Temperaturen zwischen 0° und 140° C, bevorzugt 50° bis 100° C. Bei der anschliessenden Behandlung des Reaktionsproduktes mit Wasser soll möglichst ein pH-Wert unter 9 eingestellt werden, um Salzbildung der Sulfamoylfunktion zu vermeiden.

Die Ausgangsstoffe der Formel IX erhält man durch Umsetzung eines Halogenketons der Formel XV 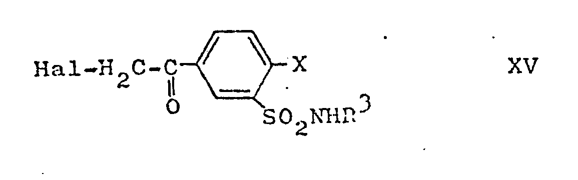 wobei Hal vorzugsweise Brom oder Jod bedeutet, mit literaturbekannten Verbindungen der Formel XVI 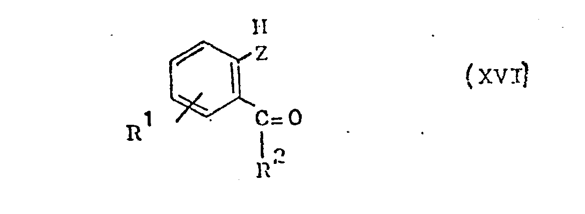 worin Z vorzugsweise S oder O bedeutet. Dabei arbeitet man unter Verwendung der bei der Cyclokondensation beschriebenen Basen in den dort genannten Lösungsmitteln, vorteilhaft unter milderen Temperaturbedingungen zwischen -10° und +60° C, vorzugsweise aber zwischen +10° und +40° C. Man kann die Herstellung von IX und deren Cyclokondensation zu I auch ohne Isolierung der Verbindungen IX in einer Eintopfreaktion ablaufen lassen.

Die erfindungsgemässen Verbindungen der Formel I, beidenen _{R}³ niederes Alkyl bedeutet, können auch durch Alkylierung der unsubstituierten Sulfamoylgruppe in üblicher Weise erhalten werden. Für die Alkylierungsreaktion verwendet man übliche Alkylierungsmittel der Formel R³-X, worin X beispielsweise für Brom, Jod, Chlor, -O-SO₂CH₃, -O-SO₂OR³ oder 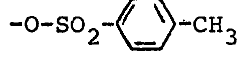 steht..

Bei der Alkylierung arbeitet man in Wasser, vorzugsweise jedoch in polaren organischen Lösungsmitteln wie einem niederen Alkohol mit 1 bis 4 C-Atomen, in Dioxan, Tetrahydrofuran, Dimethylformamid, Dimethylacetamid oder einem Mono-, Di-, Triäthylenglykolmono- oder -dimethyl- bzw. -äthyläther bei Temperaturen.zwischen -20° und +50° C, vorzugsweise zwischen +15° und +35° C, wobei man über einen Zeitraum von 5 bis 72 Stunden reägieren lässt. Als Base zur Säurebindung verwendet man vorzugsweise Karbonate, Alkoholate oder Hydroxide des Natriums oder Kaliums.

Die wichtigsten erfindungsgemässen Verbindungen sinddiejenigen der allgemeinen Formel I, in denen der SubstituentX für Brom oder Chlor, bevorzugt für Chlor, steht, R³ Wasserstoff, Methyl oder Äthyl, bevorzugt Wasserstoff, bedeutet, R² für Wasserstoff, Methyl oder Äthyl steht, R1 Wasserstoff, Chlor, Methyl oder Methoxy in Position 4 oder 5 des Heterocyclus, bevorzugt aber Wasserstoff bedeutet und Y für Sauerstoff oder Schwefel steht.

Erfindungsgemäss können ausser den in den Ausführungsbeispielen beschriebenen Substanzen auch die nachfolgend aufgeführten Verbindungen der allgemeinen Formel I dargestellt werden:
1. 3-(4-Chlor-3-sulfamoylbenzoyl)-benzo[b]furan.
2. 3-(4-Brom-3-sulfamoylbenzoyl)-benzo[b]furan.
3. 3-(4-Chlor-3-methylsulfamoylbenzoyl)-benzo[b]furan.
4. 3-(4-Chlor-3-methylsulfamoylbenzoyl)-2-methyl- benzo[b]furan.
5. 3-(4-Brom-3-sulfamoylbenzoyl)-2-methylbenzo[b]furan.
6. 2-Methyl-3-(3-sulfamoyl-4-trifluormethylbenzoyl)-benzo[b]furan.
7. 2-Äthyl-3-(4-chlor-3-sulfamoylbenzoyl-5-methoxy- benzo[b]furan.
8. 2-Äthyl-3-(3-sulfamoyl-4-trifluormethylbenzoyl)-benzo[b]furan.
9. 3-(4-Chlor-3-sulfamoylbenzoyl)-2-propylbenzo[b]furan.
10.. 3-(4-Chlor-3-sulfamoylbenzoyl)-2-isopropyl- benzo[b]furan.
11. 2-Butyl-3-(4-chlor-3-sulfamoylberizoyl)-benzo[b]furan.
12. 2-(4-Chlor-3-sulfamoylbenzoyl)-3-raethylbenzo[b]furan.
13. 2-(4-Brom-3-sulfamoylbenzoyl)-benzo[b]furan.
14. 2-(3-Sulfamoyl-4-trifluormethylbenzoyl)-benzo[b]furan.
15. 3-Äthyl-2-(4-chlor-3-sulfamoylbenzoyl)-benzo[b]furan.
16. 2-(4-Chlor-3-methylsulfamoylbenzoyl)-3-methyl- benzo[b]thiophen.
17. 2-(4-Chlor-3-propylsulfamoylbenzoyl)-3-methyl- benzo[b]thiophen.
18. 2-(4-Chlor-3-isopropylsulfamoylbenzoyl)-3-methyl- benzo[b]thiophen.
19. 2-(3-Butylsulfamoyl-4-chlorbenzoyl)-3-methyl- benzo[b]thiophen.
20. 3-Äthyl-2-(4-chlor-3-sulfamoylbenzoyl)-benzo[b]thiophen.
21. 3-Äthyl-2-(4-chlor-3-sulfamoylbenzoyl)-5-methoxy- benzo[b]thiophen.
22. 3-Methyl-2-(3-sulfamoyl-4-trifluormethylbenzoyl)-benzo[b]thiophen.
23. 3-Äthyl-2-(4-chlor-3-isopropylsulfamoylbenzoyl)-benzo[b]thiophen.
24. 3-(4-Chlor-3-sulfamoylbenzoyl)-benzo[b]thiophen.
25. 3-(4-Chlor-3-sulfamoylbenzoyl)-2-methylbenzo[b]thiophen.
26. 2-Äthyl-3-(4-chlor-3-sulfamoylbenzoyl)-benzo[b]thiophen.
27. 3-(4-Chlor-3-sulfamoylbenzoyl)-2-isopropyl- benzo[b]thiophen.
28. 3-(4-Chlor-3-methylsulfamoylbenzoyl)-2-methyl- benzo[b]thiophen.
29. 2-Äthyl-3-(4-chlor-3-methylsulfamoylbenzoyl)-benzo[b]thiophen.
30. 5-Chlor(-3-(4-chlor-3-sulfamoylbenzoyl)-2-methyl- benzo[b]thiophen.
31. 3-(4-Brom-3-sulfamoylbenzoyl)-2-methylbenzo[b]thiophen.
32. 2-Methyl-3-(3-sulfamoyl-4-trifluormethylbenzoyl)-benzo[b]thiophen.
33. 3-(3-Butyl_{ß}ulfamoyl-4-chlorbenzoyl)-2-methyl- benzo[b]thiophen.
34. 3-(4-Chlor-3-sulfamoylbenzoyl)-5-methoxy-3-methyl- benzo[b]thiophen.
35. 2-Äthyl-3-(4-brom-3-sulfamoylbenzoyl)-benzo[b]thiophen.
36. 2-(4-Chlor-3-sulfamoylbenzoyl)-indol
37. 2-(4-Chlor-3-sulfamoylbenzoyl)-3-methylindol
38. 2-(4-Chlor-3-sulfaraoylbenzoyl)-1-methylindol
39. 2-(4-Chlor-3-sulfamoylbenzoyl)-1,3-dimethylindol.
40. 1-Äthyl-2-(4-chlor-3-sulfamoylbenzoyl)-3-methylindol.
41. 3-Äthyl-2-(4-chlor-3-sulfamoylbenzoyl)-1-methylindol.
42. 3-Äthyl-2-(4-chlor-3-sulfamoylbenzoyl)-indol.
43. 2-(4-Brom-3-sulfamoylbenzoyl)-1,3-dimethylindol.
44. 2-(4-Chlor-3-sulfamoylbenzoyl)-5-methoxy-3-methylindol.
45. 5-Äthoxy-2-(4-chlor-3-sulfamoylbenzoyl)-3-methylindol.
46. 2-(4-Chlor-3-methylsulfamoylbenzoyl)-1,3-dimethylindol.
47. 2-(4-Chlor-3-propylsulfamoylbenzoyl)-1,3-dimethylindol.
48. 2-Äthyl-3-(4-chlor-3-sulfamoylbenzoyl)-indol.
49. 1-Äthyl-3-(4-chlor-3-sulfamoylbenzoyl)-2-methylindol.
50. 3-(4-Brom-3-sulfamoylbenzoyl)-2-methylindol.
51. 3-(4-Brom-3-sulfamoylbenzoyl)-1,2-dimethylindol.
52. 3-(4-Chlor-3-sulfamoylbenzoyl)-5-methoxy-2-methylindol.
53. 5-Äthoxy-3-(4-chlor-3-sulfamoylbenzoyl-1,3-dimethylindol.
54. 3-(4-Chlor-3-sulfamoylbenzoyl)-2-methyl-1-propylindol.
55. 3-(4-Chlor-3-methylsulfamoylbenzoyl)-2-methylindol.
56. 3-(4-Chlor-3-methylsulfamoylbenzoyl)-1,2-dimethylindol.
57. 1,2-Dimethyl-3-(3-sulfamoyl-4-trifluormethylbenzoyl)-indol.
58. 5-Äthoxy-3-(4-chlor-3-sulfamoylbenzoyl)-2-methylindol.
59. 2-(4-Chlor-3-sulfamoylbenzoyl)-7-methoxy- benzo[b]furan.
60. 2-(4-Chlor-3-sulfamoylbenzoyl)-5-methoxy- benzo[b]furan.
61. 2-Äthyl-3-(4-chlor-3-sulfamoylbenzoyl)-5-methoxy- benzo[b]furan.
62.. 2-Äthyl-3-(4-chlor-3-sulfamoylbenzoyl)-7-methoxy- benzo[b]furan.
63. 2-(4-Chlor-3-sulfamoylbenzoyl)-6-methoxybenzo[b]furan.
64. 2-(4-Chlor-3-methylsulfamoylbenzoyl)-6-methoxy- benzo[b]furan.
65. 2-(4-Chlor-3-sulfamoylbenzoyl)-5-methoxybenzo[b]furan.
66. 2-(4-Chlor-3-methylsulfamöylbenzoyl)-5-methoxy- benzo[b]furan.
67. 2-(4-Chlor-3-sulfamoylbenzoyl)-4-methoxy- benzo[b]furan.
68. 5-Äthoxy-2-(4-chlor-3-sulfamcylbenzoyl)-benzo[b]furan.
69. 6-Äthoxy-2-(4-chlor-3-sulfamoylbenzoyl)-benzo[b]furan.
70. 2-Äthyl-3-(4-chlor-3-sulfamoylbenzoyl)-7-methoxy- benzo[b]furan.
71. 2-Äthyl-3-(4-chlor-3-sulfamoylbenzoyl)-6-methoxy- benzo[b]furan.
72. 2-Äthyl-5-äthoxy-l-(4-chlor-3-sulfamoylbenzoyl)-benzo[b]furan.
73. 2-Äthyl-3-(4-chlor-3-methylsulfamoylbenzoyl)-7-methoxybsnzo[b]furan.

Die Verfahrensprodukte sind wertvolle Arzneimittel und verursachen eine sehr gute, den Harnsäurespiegel des Blutes senkende Wirkung, die insbesondere durch eine urikosurische Wirkung hervorgerufen wird.

Es ist bekannt, dass die meisten der zur Behandlung urikopathischer Krankheitsbilder verwendeten Arzneimittel über keine salidiuretische Wirkkomponente verfügen. Die erfindungsgemässen Verbindungen zeichnen sich dagegen durch eine erwünschte gute diuretische und saluretische Wirksamkeit aus, und sind somit den vorbekannten urikosurischen Mitteln überlegen.

Die urikosurische Wirkung der neuen Verfahrensprodukte wurde an der Oxonat behandelten Ratte in einer Einheitsdosis von 50 mg/kg per os bestimmt. Sie zeigen dabei die antiurikopathische Wirksamkeit bekannter Handelspräparate des Probencid-Typs und des Benzbromaron-Typs.

Die salidiuretische Wirkung der erfindungsgemässen Verbindungen wurde an der Ratte in einer Einheitsdosis von 50 mg/kg per os bestimmt. Sie erreichen dabei die salidiuretische Aktivität bekannter Handelspräparate wie die des Chlorthalidons. Darüber hinaus zeichnen sich die neuen Verfahrenserzeugnisse durch eine lang anhaltende Wirkungsdauer aus, wodurch die Präparate ebenfalls.zur Behandlung hypertoner Zustände am Menschen geeignet sind. Dabei kann man sie mit einem Antihypertonikum kombinieren.

Als therapeutische Zubereitung der neuen Verbindungen kommen vor allem Tabletten, Dragees, Kapseln, Suppositorien sowie auch Ampullen zur parenteralen Verabreichung (i.v., s.c. und i.m.) in Frage.

Die therapeutische Einheitsdosis liegt zwischen 5 und 1000 mg, vorzugsweise 10 bis 500 mg pro Tablette.

Diese Zubereitungen können speziell bei der Behandlung des Bluthochdrucks ausser den üblichen Füll- und Trägerstoffen noch ein Antihypertensivum, wie beispielsweise Reserpin, Hydralazin, Guanethidin, α-Methyldopa, ein ß-Sympathikolytikum oder Chloridin enthalten.

Ausserdem sind therapeutische Kombinationspräparate mit kaliumretinierenden Verbindungen, wie Aldosteronantagonisten, z.B. Spironolacton, oder Pseudoaldosterinantagonisten, wie Triamteren oder Amilorid, von Interesse. Weiterhin kommt K⁺⁻Substitution in verschiedenen Anwendungsformen, z.B. Dragees, Tabletten, Brausetabletten, Säften u.a. in Frage.

Von therapeutischem Interesse können ebenfalls Kombinationen der erfindungsgemässen Verbindungen mit einem anderen antihyperurikämisch wirksamen Mittel sein, das besonders über eine Hemmung der Xanthinoxidase zu einer Verstärkung der antiurikopathischen Effekte führt. Eine gegebenenfalls erwünschte Verstärkung der salidiuretischen Wirksamkeit kann durch Kombination der erfindungsgemässen Verbindungen mit einem Salidiuretikum erzielt werden.

In den nachfolgenden Beispielen sind die Schmelz- und Zersetzungspunkte der Ausführungsbeispiele nicht korrigiert.

### Beispiel 1:

### 2-Äthyl-3-(4-chlor-3-sulfamoylbenzoyl)-benzo[b]furan

10 g gepulvertes 4-Chlor-3-sulfamoylbenzoylchlorid, dargestellt aus 4-Chlor-3-sulfamoylbenzoesäure und Thionylchlorid (Schmp. 166° C), werden in 70 ml wasserfreies Chlorbenzol eingetragen, anschliessend 6,32 g 2-Äthyl- benzo[b]furan zugesetzt und das Reaktionsgemisch auf 0° C abgekühlt. Nach Zugabe von 11,4 g wasserfreiem Aluminiumchlorid hält man die Reaktion durch Aussenkühlung auf 5° bis 10° C, rührt sodann 5 Stunden bei 15° C und giesst das Gemisch in eine Suspension aus 200 g Eis und 10 ml konz. Salzsäure. Nach Extraktion mit Essigester und Waschen mit Wasser rührt man die organische Phase 6 Stunden mit einer verdünnten NaHCO₃-Lösung vom pH 8,5, trocknet über Magnesiumsulfat und erhält nach Verdampfen ein hellgelbes bis farbloses viskoses öl, das unter Petroläther zur Kristallisation kommt. Farblose Kristalle, Schmp. 170° - 172° C (aus wenig Methanol).

### Beispiel 2:

### 2-Äthyl-3-(3-butylsulfamoylbenzoyl)-benzo[b]furan

erhält man analog der in Beispiel 1 angegebenen Vorschrift aus 10 g 3-Butylsulfamoyl-4-chlorbenzoylchlorid und 5,1 g 2-Äthylbenzo[b]furan in Gegenwart von 9,4 g Aluminiumchlorid als farbloses bis schwach gelbes viskoses öl.

### Beispiel 3:

### 2-Äthyl-3-(4-chlor-3-sulfamoylbenzoyl)-5-methyl- benzo[b]furan

erhält man analog der in Beispiel 1 angegebenen Vorschrift aus 10 g 4-Chlor-3-sulfamoylbenzoylchlorid und 6,2 g 2- Äthyl-5-methylbenzo[b]furan in Gegenwart von 11,4 g Aluminiumchlorid. Farblose Kristalle vom Schmp. 147° - 150° C (aus Methanol).

### Beispiel 4:

### 2-Äthyl-5-chlor-3-(4-chlor-3-sulfamoylbenzoyl)-benzo[b]furan

erhält man analog der in Beispiel 1 angegebenen Vorschrift aus 10 g 4-Chlor-3-sulfamoylbenzoylchlorid und 7,06 g 2- Äthyl-5-chlorbenzo[b]furan in Gegenwart von 11,4 g Aluminiumchlorid. Farblose Kristalle, Schmp. 130° - 133° C.

### Beispiel 5:

### 3-(4-Chlor-3-sulfamoylbenzoyl)-2-methylbenzo[b]furan

erhält man analog der in Beispiel 1 angegebenen Vorschrift aus 10,1 g 4-Chlor-3-sulfamoylbenzoylchlorid und 5,8 g 2-Methylbenzo[b]furan in Gegenwart von 11,4 g Aluminium- .chlorid. Nach Verdampfen des Extraktionsmittels rührt man den Rückstand unter Diisopropyläther und filtriert den Feststoff ab. Farblose Kristalle, Schmp. 183° C (aus Methanol).

### Beispiel 6:

### 2-Äthyl-3-(4-chlor-3-methylsulfamoylbenzoyl)-benzo[b]furan

erhält man analog der in Beispiel 1 angegebenen Vorschrift aus 10,7 g 4-Chlor-3-methylsulfamoylbenzoylchlorid und 6,4 g 2-Äthylbenzo[b]furan als amorphen Feststoff vom Erweichungspunkt 63° C.

### Beispiel 7:

### 2-Äthyl-3-(4-brom-3-sulfamoylbenzoyl)-benzo[b]furan

erhält nian analog der in Beispiel 1 angegebenen Vorschrift aus 10,9 g 4-Brom-3-sulfamoylbenzoylchlorid und 6 g 2-Äthyl- benzo[b]furan in Gegenwart von 11,7 g Aluminiumchlorid. Farblose Kristalle, Schmp. 202° - 206° C (Äther).

### Beispiel 8:

### 2-(4-Chlor-3-sulfamoylbenzoyl)-benzo[b]furan

### a) 2-(4-Chlor-3-nitrobenzoyl)-benzo[b]furan

Zu einer Natriummethylatlösung, dargestellt aus 3,13 g Natrium und 170 ml Methanol,tropft man unter N₂-Schutz rasch 16,6 g Salicylaldehyd, destilliert sodann das Lösungsmittel ab und schlämmt den Rückstand in 150 ml wasserfreiem Toluol auf. Zu dem Gemisch gibt man 38 g 2--Brom-4'-chlor-3'-nitroacetoplaenon in 100 ml Toluol unter Rührung zu und kocht anschliessend 3 Stunden am Rückflusskühler. Nach dem Stehenlassen bei Raumtemperatur über Nacht destilliert man das Lösungsmittel ab und kristallisiert den Rückstand aus Äthanol um. Hellgelbe Kristalle, Schmp. 132° C.

### _{b}) 2-(3-Amino-4-chlorbenzoyl)-benzo[b]furan

37 g 2-(4-Chlor-3-nitrobenzoyl)-benzo[b]furan werden unter Rührung in einer Mischung aus 500 ml 50%iger wässriger Essigsäure, 250 ml Äthanol und 90 g gepulverter Nickel-Aluminium-Legierung (1 : 1) 4 Stunden am Rückflusskühler gekocht. Nach Filtration des Metallpulvers destilliert man das Lösungsmittel ab, versetzt den - Rückstand mit Wasser und extrahiert mit Essigsäureäthyl-ester. Nach Vertreiben des Lösungsmittels erhält man farblose Kristalle vom Schmp. 137° C (aus Isopropanol);

### c) 2-(4-Chlor-3-chlorsulfonylbenzoyl)-benzo[b]furan

3 g 2-(3-Amine-4-chlorbenzoyl)-benzo[b]furan werden in 10 ml Eisessig und 5 ml H₂O aufgeschlämmt-und sodann mit 10 ml konz. Salzsäure versetzt. Bei 0° bis 5° C tropft man unter Rührung eine Lösung von 1 g Natriumnitrit in 4 ml Wasser unter die Oberfläche. Das Reaktionsgemisch wird sodann portionsweise in eine Mischung aus 2,3 g CuCl₂ x 2 H₂0 in 70 ml SO₂-gesättigte Eisessiglösung eingetragen und nach 20 Minuten Rührung das Volumen mit Wasser verdoppelt. Man rührt 45 Minuten nach, filtriert die Kristalle ab und trocknet.
Schmp. 131° C (Zers.)

### d) 2-(4-Chlor-3-sulfamoylbenzoyl)-benzo[b]furan

3,3 g 2-(4-Chlor-3-chlorsulforsylbenzoyl)-benzo[b]furan werden in 19 ml 25%ige wässrige Ammoniaklösung eingetragen und nach Stehenlassen über Nacht die Flüssigkeit abdestilliert. Nach Zugabe von Wasser stellt man mit verdünnter HC1 auf pH 6, rührt 30 Minuten nach und kristallisiert aus wenig Eisessig. Farblose Kristalle, Schmp. 181° C.

### Beispiel 9:

### 3-(4-Chlor-3-sulfamoylbenzoyl)

5,1 g gepulvertes 4-Chlor-3-sulfamoylbenzoylchlorid wer- den in eine Lösung aus 3 g Benzo[b]thiophen in 50 ml wasserfreiem Toluol eingetragen und anschliessend unter Rührung mit 12 g Titantetrachlorid versetzt. Man rührt 15 Min. bei Raumtemperatur, erhitzt sodann etwa 15 Minuten zum Sieden, kühlt ab und giesst auf eine Wasser-Eis-Suspension. Nun extrahiert man mit 70 ml Essigester, trennt die organische Phase ab und rührt diese 5 Stunden mit einer wässrigen Natriumbicarbonatlösung vom pH 8,5. Nach Abtrennen der organischen Phase und deren Trocknung über Magnesiumsulfat wird das Lösungsmittel abdestilliert und der Rückstand unter Toluol zur Kristallisation gebracht. Schmp. 154° - 156° C.

### Beispiel 10_{:}

### 3-(4-Chlor-3-sulfamoylbenzoyl)-2-methylindol

a) 2,5 g 4-Chlor-3-sulfamoylbenzoylchlorid werden in 25 ml Dichloräthan aufgeschlämmt und portionsweise unter Rührung zu einer Suspension von 2,7 g Aluminiumchlorid in 25 ml Dichloräthan gegeben, wobei die Temperatur durch Aussenkühlung zwischen -10° C und 0° C gehalten wird. Die erhaltene klare Lösung rührt man 1 Stunde bei +5° C, erwärmt sodann auf 30° C und gibt sodann eine Lösung von 1,3 g 2-Methylindol in 25 ml Dichloräthan zu, wobei die Temperatur zwischen 30° und 40° C gehalten wird. Die Reaktionsmischung wird auf etwa 10° C gekühlt, sodann mit Eiswasser zersetzt und zweimal mit Essigester extrahiert. Die vereinigten organischen Phasen rührt man 8 Stunden mit wässriger Natriumbicarbonatlösung vom pH 8 - 9, wäscht diese mit Wasser, trocknet über Natriumsulfat und vertreibt das Lösungsmittel unter vermindertem Druck. Der Rückstand wird mit Diisopropyläther gerührt und der kristalline Feststoff abfiltriert. Schmp. 258° - 260° C.
b) 3,1 g 4-Chlor-3-sulfamoylbenzoesäure-N,N-dimethylamid, dargestellt aus 4-Chlor-3-sulfamoylbenzoylchlorid und wässriger Dimethylaminlösung (Schmp. 143° C) werden zusammen mit 0,8 g 2-Methylindol und 0,7 ml Phosphoroxichlorid unter Rührung und Ausschluss von Luftfeuchtigkeit über 2 Stunden auf 80° C erhitzt. Man stellt mit 2N NaOH alkalisch, rührt 40 Stunden bei Raumtemperatur, bringt sodann auf pH 7 bis 8 und filtriert die Kristalle ab. Schmp. 257° - 260° C.
c) In eine Lösung von 10 g 4-Chlor-3-sulfamoylbenonitril, dargestellt durch Rückfluss von 4-Chlor-3-sulfamoyl- benzamid in Phosphoroxichlorid (Schmp. 199° C) und 6,06 g 2₋Methylindol in 80 ml wasserfreien Diäthylenglykoldimethyläther leitet man 5 Stunden bei 15° - 20° C einen Strom Chlorwasserstoffgas ein und lässt das Gemisch 72 Stunden bei 10° - 15° C stehen. Durch Eingies- .sen des Reaktionsgemisches in Essigester scheidet sich das 4'-Chlor-3'-sulfamoylphenyl-2-methyl-3-indolyl- ketonimin-hydrochlorid kristallin ab. Schmp. 320° C. 9 g Ketonimin-hydrochlorid werden in 100 ml heissem Wasser gelöst, nach Zugabe von wässrigem Ammoniak unter Rührung bei 50° - 60° C hydrolysiert und das kristalline 3-(4-Chlor-3-sulfamoylbenzoyl)-2-methylindol abfiltriert. Schmp. 252° - 257° C.
d) Zu einer Lösung von Methylmagnesiumjodid, dargestellt aus 3,28 g Magnesiumspänen in 80 ml wasserfreiem Äther und 22,5 g Methyljodid, fügt man rasch eine Lösung von 17 g 2-Methylindol in 200 ml Tetrahydrofuran und erhitzt ca. 15 Minuten zum Rückfluss. Sodann lässt man rasch eine Lösung von 15,75 g 4-Chlor-3-sulfamoylben- zoylchlorid in 100 ml Tetrahydrofuran zufliessen, erhitzt weitere 14 Stunden am Rückflusskühler, kühlt ab und giesst in eine Lösung aus 20 g Ammoniumchlorid in 200 ml Wasser. Nach Zugabe von 350 ml Essigester rührt man 10 Minuten, filtriert das in homogene Gemisch über eine Klärschicht, trennt sodann die organische Phase ab und trocknet nach einmaligem Waschen mit Wasser über Magnesiumsulfat. Nach dem Verdampfen des Lösungsmittels suspendiert man den kristallinen Rückstand in Diisopropyläther und filtriert die Kristalle ab. Schmp. 255° - 259° C.
e) Die nach den Methoden a) bis d) dargestellten Proben des 3-(4-Chlor-3-sulfamoylbenzoyl)-2-methylindols zeigen identische IR-Spektren und depressionslose Mischschmelzpunkte.

### Beispiel 11:

### 3-(4-Chlor-3-methylsulfamoylbenzoyl)-2-methylindol

erhält man analog der in Beispiel 10 a) angegebenen Vorschrift aus 2,68 g 4-Chlor-3-methylsulfamoylbenzoyl- chlorid und 1,3 g 2-Methylindol in Gegenwart von 2,7 g Aluminiumchlorid in Dichloräthan. Farblose Kristalle, Schmp. 246° C.

### Beispiel 12: .

### 3-(4-Chlor-3-sulfamoylbenzoyl)-2,5-dimethylindol

erhält man analog der in Beispiel 10 a) angegebenen Vorschrift aus 10 g 4-Chlor-3-sulfamoylbenzoylchlorid und 7,8 g 2,5-Dimethylindol in Dichloräthan in Gegenwart von 12 g Aluminiumchlorid. Schmp. 248° - 250° C.

### Beispiel 13:

### 3-(4-Chlor-3-sulfamoylbenzoyl)-1,2-dimethylindol

erhält man analog der in Beispiel 10 a) angegebenen Vorschrift aus 10 g 4-Chlor-3-sulfamoylbenzoylchlorid und 6,53 g 1,2-Dimethylindol in Dichloräthan in Gegenwart von 12 g Aluminiumchlorid. Nach Behandeln mit Isopropanol erhält man Kristalle vom Schmp. 247° - 249° C.

### Beispiel 14:

### 2-(4-Chlor-3-sulfamoylberzoyl)-benzo[b]furan

I a) 8 g 4-Chlor-3-sulfamoylbenzonitril und 12 g Nickel-Aluminium-Legierung (1 : 1) werden in 120 ml 75%iger wässriger Ameisensäure 2 Stunden am Rückflusskühler gekocht, sodann heiss abfiltriert und das unumgesetzte Metallpulver mehrfach mit heissem Methanol nachgewaschen. Nach dem Einengen versetzt man mit Wasser und filtriert den kristallinen 4-Chlor-3-sulfamoyl- benzaldehyd (Schmp. 162° - 164° C) ab.
   .b) 6 g (0,03 Mol) 2-Brombenzo[b[furan. werder in 20 ml Diäthyläther gelöst und auf einmal zu einer auf -70° abgekühlten Lösung von 0,033 Mol n-Butyllithium in 150 ml wasserfreiem Tetrahydrofuran gegeben. Man rührt etwa 5 Minuten nach und gibt nun in kleinen Portionen eine Mischung von 2,2 g (0,01 Mol) 4-Chlor-3-sulfamoyl- benzaldehyd in 30 ml wasserfreiem Tetrahydrofuran zu, wobei das Reaktionsgemisch bei -40° bis -70° C gehalten wird. Man rührt 20 Min. bei -40° C, rührt sodann 30 Stunden bei Zimmertemperatur und 6 weitere Stunden bei +50° C und behandelt das Gemisch anschliessend unter Eiskühlung mit 10 ml einer gesättigten Ammoniumchloridlösung. Der Niederschlag wird abfiltriert, mehrfach mit Essigester gewaschen, die vereinigten organischen Phasen über Magnesiumsulfat getrocknet und das Lösungsmittel unter vermindertem Druck verdampft. Farblose Kristalle, Zersetzungspunkt 148° C.
   c) 1,69 g (0,05 Mol) 4-Chlor-3-sulfamoylphen^{y}l-2'-benzo[b]furan-carbinol werden in 40 ml Acetonitril gelöst und nach Zugabe von 6 g aktivem Mangandioxid 60 Stunden bei Raumtemperatur gerührt. Man filtriert das anorganische Material ab, wäscht einmal mit Acetonitril nach, engt unter vermindertem Druck ein und kristalisiert den Rückstand aus wenig Eisessig um. Farblose Kristalle, Schmp. 170° - 172° C.
II) Zu einer wie unter I b) dargestellten Lösung von 2-Benzo[b]furyllithium in Tetrahydrofuran fügt man eine Mischung aus 2,16 g 4-Chlor-3-sulfamoylbenzonitril in 50 ml absol. Tetrahydrofuran und erhitzt unter Stickstoffschutz und guter Rührung 18 Stunden amRückflusskühler. Sodann wird das Lösungsmittel unter vermindertem Druck abdestilliert, der Rückstand mit Eiswasser versetzt und mit 2N HCl sauer gestellt. Man rührt 2 Stunden bei Raumtemperatur, extrahiert das öl mit 100 ml Essigester, wäscht die organische Phase zweimal mit Wasser und trocknet über Natriumsulfat. Nach dem Abdestillieren des Lösungsmittels unter vermindertem Druck erhält man ein schwach gelbes Öl, das in 20%iger wässriger KOH 20 Minuten bei Raumtemperatur gerührt und sodann in eine gute gerührte, gesättigte, wässrige Ammoniumchloridlösung getropft. Man saugt ab und kristallisiert aus wenig Eisessig um. Farblose Kristalle, Schmp. 167° - 170° C.
III, 4 2-Brom-4'-chlor-3'-sulfamoylacetophenon werden zusammen mit 1,63 g Salizylaldehyd in Gegenwart von 2,9 g unter wasserfreien Bedingungen gemahlenen Kaliumcarbonat 2 Stunden unter Rührung und unter Ausschluss von Feuchtigkeit in 50 ml wasserfreiem Dimethylformamid auf 80° C gehalten. Nach dem Abkühlen giesst man in ein Gemisch aus Eiswasser und überschüssiger Salzsäure, extrahiert mit Essigester und trocknet über Natriumsulfat. Nach Abdestillieren des Lösungsmittels bringt man unter Diisopropyläther und unter Isopropanol zur Kristallisation, Schmp. 152° - 156° C. Umkristallisation aus Eisessig nach Klärung mit Aktivkohle liefert farblose Kristalle vom Schmp. 167° - 170° C.

### Beispiel 15:

### 2-(4-Chlor-3-sulfamoylbenzoyl)-3-methyl-benzo[b]thiophen

erhält man analog der in Beispiel 1 angegebenen Vorschrift aus 2,5 g 4-Chlor-3-sulfamoylbenzoylchlorid, 1,6 g 3-Methyl-benzo[b]thiophen und 3 g Aluminiumchlorid in 50 ml Chlorbenzol. Farblose Kristalle, Schmp. 210° C.

### Beispiel 16:

### 2-(4-Chlor-3-sulfamoylbenzoyl)-3-methylindol

erhält man analog der in Beispiel 10 a) angegebenen Vorschrift aus 10,0 g 4-Chlor-3-sulfamoylbenzoylchlorid, 5,1 g 3-Methylindol in Gegenwart von 10,4 g Aluminiumchlorid. Farblose Kristalle aus Isopropanol, Schmp. 205° C.

### Beispiel 17:

### 3-(4-Chlor-3-sulfamoylbenzoyl)-2-methylbenzo[b]furan

a) 3-(4-Chlor-3-nitrobenzoyl)-2-methylbenzo/b7furan 4,4 g 4-Chlor-3-nitrobenzoylchlorid und 2,6 g 2-Methyl- benzo[b]furan werden in 50 ml Chlorbenzol gelöst, auf 0° C gekühlt, und nach Zugabe von 4 g wasserfreiem Aluminiumchlorid 1 Stunde bei 0° bis 5° C gerührt. Nach dem Stehenlassen über Nacht bei Raumtemperatur giesst man auf Eiswasser, extrahiert mit mehrfach Essigester, wäscht die vereinigten organischen Phasen mit Wasser und rührt intensiv mit einer wässrigen NaHCO₃-Lösung (pH 8 - 9) bis zum Verschwinden der geringen Anteile an 4-Chlor-3-nitrobenzoylchlorid. Man trocknet über Natriumsulfat, destilliert das Lösungsmittel unter vermindertem Druck ab und bringt den Rückstand unter Äther zur Kristallisation. Schmp. 122° C.
b) 3-(3-Amino-4-chlorbenzoyl)-2-methylbenzo[b]furan erhält man analog der in Beispiel 8 b) angegebenen Vorschrift aus 3,2 g 3-(4-Chlor-3-nitrobenzoyl)-2-methyl- benzo[b]furan mit 9,6 g Nickel-Aluminium-Legierung (1 : 1). Schmp. 103° C.
c) 3-(4-Chlor-3-chlorsulfamoylbenzoyl)-2-methyl- benzo[b]furan
   erhält man analog der in Beispiel 8 c) angegebenen Vorschrift aus 3-(3-Amino-4-chlorbenzoyl)-2-methyl- benzo[b]furan. Farblose Kristalle, Schmp. 138° C.
d) 3-(4-Chlor-3-sulfamoylbenzoyl)-2-methylbenzo[b]furan erhält man durch Umsetzung von 2,3 g 3-(4-Chlor-3- chlorsulfonylbenzoyl)-2-methylbenzo[b]furan in einer Mischung aus 20 ml Methanol und 10 ml wasserfreiem Ammoniak. Nach dem Stehenlassen über Nacht destilliert man das Lösungsmittel ab, nimmt den Rückstand in Wasser auf und filtriert ab. Farblose Kristalle aus wenig Äthanol, Schmp. 180° - 183° C.

### Beispiel 18:

### 2-(4-Chlor-3-sulfamoylbenzoyl)-7-methoxybenzo[b]furan

a) 2-(4-Chlor-3-nitrobenzoyl)-7-methoxybenzo[b]furan 2 g KOH werden in 50 ml Methanol gelöst, versetzt sodann mit einer Lösung aus 5,4 g 2-Hydroxy-3-methoxybenzaldehyd und anschliessend mit einer Suspension aus 10 g 2-Brom-4'-chlor-3'-nitroacetophenon in 75 ml Methanol. Man kocht 6 Stunden am Rückflusskühler, rührt über Nacht bei Raumtemperatur und saugt die Kristalle ab. Schmp. 136° C (aus Essigester).
b) 2-(3-Amino-4-chlorbenzoyl)-7-methoxyberzo[b]furan erhält man analog der in Beispiel 8 b) angegebenen Vorschrift aus 2,2 g 2-(4-chlor-3-nitrobenzoyl-7-methoxy⁻ benzo[b]furan und 6,6 g Nickel-Aluminium-Legierung (1 : 1). Farblose Kristalle, Schmp. 180° C(aus Äthanol).
c) 2-(4-Chlor-3-chlorsulfonylbenzoyl)-7-methoxy- benzo[b]furan
   erhält man analog der in Beispiel 8 c) angegebenen Vorschrift aus 2-(3-Amino-4-chlorbenzoyl)-7-methoxy- benzo[b]furan. Schmp. 143° - 145° C.
d) 2-(4-Chlor-3-sulfamoylbenzoyl)-7-methoxy- benzo[b]furan
   erhält man analog der in Beispiel 8 d) angegebenen Vorschrift aus 2-(4-Chlor-3-chlorsulfamoylbenzoyl)-7-methoxybenzo[b]furan und 25%iger wässriger Ammoniaklösung. Schmp. 167° - 171° C.

### Beispiel 19:

### 3-(4-Chlor-3-methylsulfamoylbenzoyl)-2-methyl- benzo[b]furan

erhält man durch Behandlung von 4,5 g 3-(4-Chlor-3-chlor- sulfonylbenzoyl)-2-methylbenzo[b]furan mit einem Gemisch aus 20 ml Methanol und 30 ml 40%iger wässriger Methylaminlösung über 12 Stunden bei Raumtemperatur, anschliessendem Abdampfen des Lösungsmittels, Zugabe von Wasser und Filtration der Kristalle. Schmp. 146° C.

### Beispiel 20;

### 3-(4-Chlor-3-sulfamoylbenzoyl)-2-methylbenzo[b]thiophen

erhalt man analog der in Beispiel 10 a) angegebenen Vorschrift aus 4-Chlor-3-sulfamoylbenzoylchlorid und 2-Methylbenzo[b]thiophen in Dichloräthan in Gegenwart von Aluminiumchlorid.

## Patentansprüche

1. Verbindungen der allgemeinen Formel I 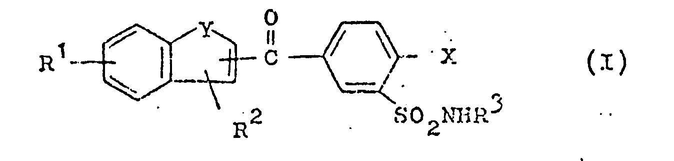 worin R¹ Wasserstoff, Halogen, Alkyl mit 1 bis 3 C-Atomen,Methoxy oder Äthoxy, R² und R³ gleich oder verschieden sein können und Wasserstoff oder Alkyl mit 1 bis 4 C-Atomen, X ein Halogenatom, Methyl oder Trifluormethyl und Y Sauerstoff, Schwefel oder NR4 bedeuten, wobei R⁴ für Wasserstoff oder Alkyl mit 1 bis 4 C-Atomen steht.

2. 2-Äthyl-3-(4-chlor-3-sulfamoylbenzoyl)-benzo[b]furan.

3. 3-(4-Chlor-3-sulfamoylbenzoyl)-2-methylbenzo[b]furan.

4. 3-(4-Chlor-3-sulfamoylbenzoyl)-2-methylindol.

5. 3-(4-Chlor-3-merhylsulfamoylbenzoyl)-2-methylindol.

6. 2-(4-Chlor-3-sulfamoylbenzoyl)-benzo[b]furan.

7. 2-(4-Chlor-3-sulfamoylbenzoyl)-7-methoxy-benzo[b]furan.

8. 3-(4-Chlor-3-sulfamoylbenzoyl)-2-methylbenzo[b]thiophen.

9. 2-(4-Chlor-3-sulfamoylbenzoyl)-3-methylbenzo[b]thiophen.

10. Verfahren zur Herstellung von Verbindungen gemäss Anspruch 1, dadurch gekennzeichnet, dass man
a) Verbindungen der allgemeinen Formel II 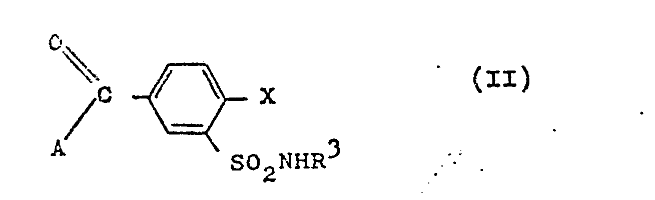 worin R³ und X die angegebene Bedeutung besitzen und A für Halogen, Acyloxy, Hydroxy oder NR₂ steht und R Wasserstoff, niederes Alkyl oder Phenyl bedentet, mit einer heterocyclischen Verbindung der allgemeinen Formel III, 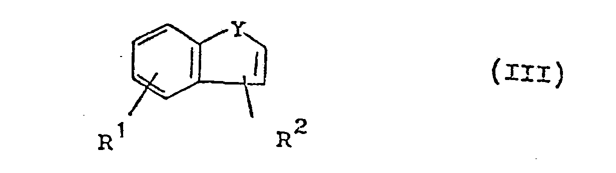 worin R¹, R² und Y die angegebene Bedeutung besitzen, in Gegenwart einer Lewis-Säure oder Protonensäure umsetzt,
b) Verbindungen der allgemeinen Formel IV 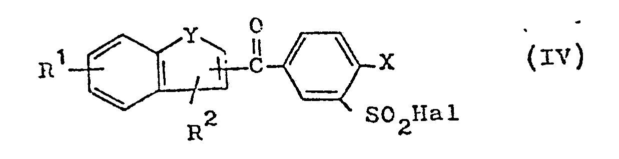 worin R¹, R², X und Y die angegebene Bedeutung besitzen und Hal für Halogen steht, mit einem Amin der allgemeinen Formel V 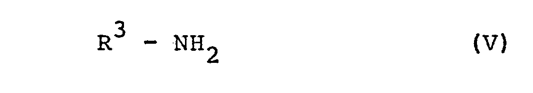 worin R3 die angegebene Bedeutung hat, umsetzt oder.
c) Verbindungen der allgemeinen Formel VI 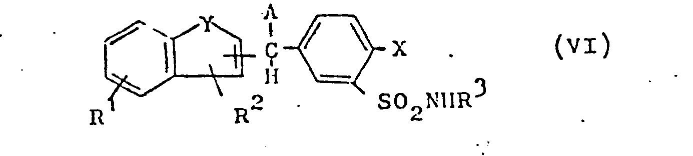 worin R¹ bis _{R}³, A, X und Y die angegebene Bedeutung besitzen, mit einem Oxidationsmittel behandelt,
d) Verbindungen der allgemeinen Formel VII 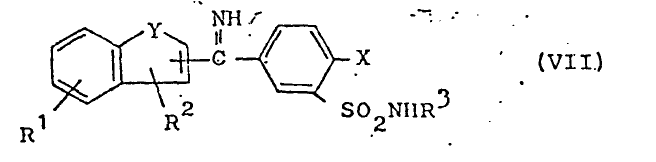 worin R¹ bis R^{3,} X und Y die obige Bedeutung haben, gegebenenfalls in Form ihrer Säureadditionssalze der Hydrolyse unterwirft,
e) Verbindungen der allgemeinen Formel VIII 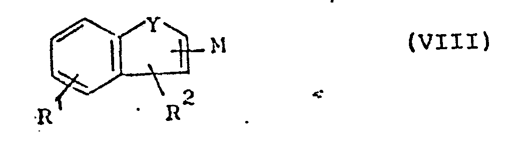 worin R¹ bis R² die angegebene Bedeutung besitzen, M für Li, MgHal, CdHal, HgHal, of HgOCOCH₃ steht, und Y neben der angegebenen Bedeutung auch diejenige von NM besitzen kann,
mit Verbindungen der allgemeinen Formel II umsetzt, worin A neben der angegebenen Bedeutung auch für einen 2-Mercaptopyridylrest 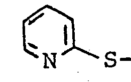 stehen kann, oder
f) Verbindungen der allgemeinen Formel IX 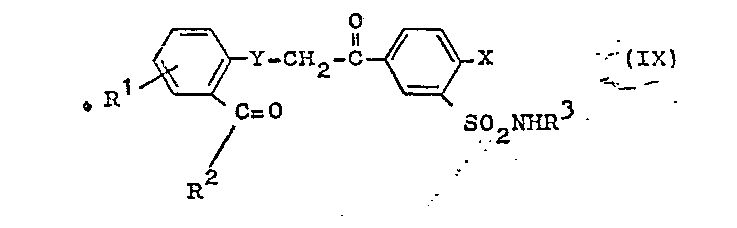 cyclisiert und gegebenenfalls die nach a) bis f) erhaltenen Verbindungen der allgemeinen Formel I,worin R³ Wasserstoff bedeutet, anschliessend alkyliert.

11. Pharmazeutische Präparate mit urikosurischer, hypourikämischer und salidiuretischer Wirkung, bestehend aus bzw. enthaltend eine Verbindung gemäss Anspruch 1.

12. Verfahren zur Herstellung pharmazeutischer Präparate mit urikosurischer, hypourikämischer und salidiuretischer Wirkung, dadurch gekennzeichnet, dass man eine Verbindung gemäss Anspruch 1 gegebenenfalls mit pharmazeutischen Trägern und/oder Stabilisatoren in eine für therapeutische Zwecke geeignete Anwendungsform bringt.

13. Verwendung von Verbindungen der Formal I zur Senkung des Harnsäurespiegels des Blutes und zur Bekämpfung des Bluthochdrucks.
